# EUROPEAN PATENT APPLICATION

(11) **EP 4 691 234 A1**
(43) Date of publication of application: **11.02.2026**
(21) Application number: 24778728.6
(22) Date of filing: 09.02.2024
(51) Int. Cl.: A01K 67/027, C12N 15/57, C12N 15/62

(54) **NON-HUMAN ANIMAL, KIT, AND METHOD FOR PRODUCING REPLACEMENT ORGAN**

(30) Priority: 28.03.2023 JP 2023051810
(71) Applicant: The Jikei University, Tokyo 105-8461 (JP)
(72) Inventor: YAMANAKA Shuichiro, Tokyo 105-8461 (JP); YOKOO Takashi, Tokyo 105-8461 (JP); MATSUI Kenji, Tokyo 105-8461 (JP)
(74) Representative: Acapo Onsagers AS
(86) International application number: PCT/JP2024/004463
(87) International publication number: WO 2024/202583

(57) **Abstract**

A non-human animal including a system configured to kill a target cell in vivo, in which the target cell includes a chromosome having a cell-specific promoter and an exogenous caspase 9 gene in which expression is inducible downstream of the cell-specific promoter, or a chromosome having a high-expression promoter and an exogenous caspase 9 gene in which expression is inducible downstream of the high-expression promoter.

## Description

### TECHNICAL FIELD

The present invention relates to a non-human animal, a kit, and a method for producing a replacement organ.

### BACKGROUND ART

A system that induces death of target cells by a drug has been widely used for research on intercellular interactions at the organismal level, research on disease models, research on malignant tumors, and the like. For example, a target cell-specific inducible cell death system using a herpes virus-derived thymidine kinase gene (HSV-TK) has been developed, and models such as a liver injury model have been produced (see Non Patent Document 1).

However, since the HSV-TK system cannot be applied to non-dividing cells and the HSV-TK system has immunogenicity, the development of other systems has been explored. Therefore, an inducible death system using diphtheria toxin has been developed, and the results have attracted because the inducible death effect on non-dividing cells has been confirmed (see Non Patent Document 2).

### Citation List

### Non Patent Documents

Non Patent Document 1: Bonini C, Ferrari G, Verzeletti S, Servida P, Zappone E, Ruggieri L, Ponzoni M, Rossini S, Mavilio F, Traversari C, Bordignon C. HSV-TK gene transfer into donor lymphocytes for control of allogeneic graft-versus-leukemia. Science. 1997 Jun 13;276(5319):1719-24. doi: 10.1126/science.276.5319.1719. PMID: 9180086.
Non Patent Document 2: Saito M, Iwawaki T, Taya C, Yonekawa H, Noda M, Inui Y, Mekada E, Kimata Y, Tsuru A, Kohno K. Diphtheria toxin receptor-mediated conditional and targeted cell ablation in transgenic mice. Nat Biotechnol. 2001 Aug;19(8):746-50. doi: 10.1038/90795. PMID: 11479567.

### SUMMARY OF INVENTION

### Technical Problem

However, since the diphtheria toxin used as the inducer has a damaging effect on human cells, it has not been usable for human cells.

An object of the present invention is to provide a non-human animal, a kit, and a method for producing a replacement organ, in which non-target human cells are not affected by the toxicity of the inducer, target cells are removed with high efficiency, and target cell death is rapidly induced.

### Solution to Problem

The present invention includes the following aspects.
[1] A non-human animal including a system configured to kill a target cell in vivo, in which the target cell includes a chromosome having a cell-specific promoter and an exogenous caspase 9 gene in which expression is inducible downstream of the cell-specific promoter, or a chromosome having a high-expression promoter and an exogenous caspase 9 gene in which expression is inducible downstream of the high-expression promoter.
[2] The non-human animal according to [1], in which the exogenous caspase 9 gene is a gene encoding a fusion protein including a dimerization domain and caspase 9.
[3] The non-human animal according to [1], in which the non-human animal includes, in a chromosome, a gene encoding a site-specific recombinase, and the non-human animal has the chromosome containing, upstream of the exogenous caspase 9 gene, a site-specific recombinase recognition sequence, a transcription termination sequence, and a site-specific recombinase recognition sequence in this order from a 5' side.
[4] A kit including the non-human animal according to [2] and a chemical inducer of dimerization.
[5] The kit according to [4], further including an apoptosis-promoting agent.
[6] A method for producing a replacement organ, including a inducing step of inducing expression of the exogenous caspase 9 gene in the non-human animal according to any one of [1] to [3], a killing step of killing a target cell of the non-human animal by a caspase 9 protein for which the expression has been induced, and an injection step of injecting a human progenitor cell of the same type as the killed target cell into a site in the non-human animal in which the target cell has been killed.
[7] The method for producing a replacement organ according to [6], further including an administration step of administering an apoptosis-promoting agent.

### Advantageous Effects of Invention

According to the present invention, it is possible to provide a non-human animal, a kit, and a method for producing a replacement organ, in which non-target human cells are not affected by the toxicity of the inducer, target cells are removed with high efficiency, and target cell death is rapidly induced.

### BRIEF DESCRIPTION OF DRAWINGS

[FIG. 1(A)] A diagram showing a construct of a Six2-iCaspase9-tdTomato mouse.
[FIG. 1(B)] A schematic diagram of a mechanism of apoptosis by activation of Caspase 9.
[FIG. 2] Bright-field and fluorescence images of a fetus and a fetal kidney of a Six2- iCaspase9-tdTomato mouse. Expression of tdTomato was observed in the Six2 expression domain.
[FIG. 3] An immunostained image of a kidney organoid prepared from renal progenitor cells extracted from a fetal kidney of a Six2- iCaspase9 mouse. The left image shows the condition without CID administration and the right image shows the condition with CID administration. Induced cell death of nephron progenitor cells was observed under the condition with CID administration.
[FIG. 4] A fluorescence image of an organ-cultured kidney excised from a fetus of a Six2-iCaspase9 mouse. The removal of nephron progenitor cells by the CID administration (lower) was also confirmed in the organ culture.
[FIG. 5] An immunostained image on day 3 in FIG. 4. In the condition with CID administration (right), the removal of Six2-positive nephron progenitor cells was confirmed.
[FIG. 6] An image showing the result of analyzing fetal mouse kidney 48 hours after intraperitoneally administration of AP20187 (CID) to a neonatal Six2-iCaspase9-tdTomato mouse In the upper of the CID administration group, the removal of Six2-positive nephron progenitor cells in the neonatal kidney was observed.
[FIG. 7] An immunostained image in FIG. 6. In the upper of the CID administration group, the removal of Six2-positive nephron progenitor cells in the neonatal kidney was observed.
[FIG. 8] The results of apoptosis evaluation by TUNEL staining. At 12 hours after administration (upper left), TUNEL positivity was observed in co-localization with Six2-positive nephron progenitor cells, indicating early induction of apoptosis.
[FIG. 9] The results of genotyping of the transgenic mouse.
[FIG. 10] The results of culturing kidneys collected from E13.5 fetuses of double allele knock-in (homozygous) mice with CID addition.
[FIG. 10(A)] Fluorescence images of tdtomato.
[FIG. 10(B)] Immunostained images of a nephron progenitor cell.
[FIG. 10(C)] The quantification results of FIG. 10(B).
[FIG. 11] The results of culturing kidneys collected from E13.5 fetuses of single allele knock-in (heterozygous) mice with CID and/or AT406 addition.
[FIG. 11(A)] Fluorescence images of tdtomato.
[FIG. 11(B)] Immunostained images of a nephron progenitor cell.
[FIG. 11(C)] The quantification results of FIG. 11(B).
[FIG. 12] The quantification results of tdtomato in a homozygous fetal kidney and a heterozygous fetal kidney.
[FIG. 13] The results of subcutaneously administering CID to homozygous neonatal mice.
[FIG. 13(A)] Fluorescence images of tdtomato.
[FIG. 13(B)] Immunostained images of a nephron progenitor cell.
[FIG. 14] The results of the administration of CID and AT406 in combination to heterozygous neonatal mice.
[FIG. 14(A)] Fluorescence images of tdtomato.
[FIG. 14(B)] Immunostained images of a nephron progenitor cell.
[FIG. 15] A diagram showing a scheme in which CID is intraperitoneally administered to a pregnant dam carrying homozygous fetuses.
[FIG. 16(A)] The results of confirming sizes of neonatal kidneys, which have been administered with CID at different time points, by fluorescence images of tdtomato.
[FIG. 16(B)] The quantification results of a kidney size (major axis) based on FIG. 16(A).
[FIG. 17] An immunostained image of nephron progenitor cells with and without CID administration.
[FIG. 18(A)] An immunostained image of glomeruli which have been administered with CID at different time points.
[FIG. 18(B)] The quantification results of the number of glomeruli based on FIG. 18(A).
[FIG. 19] The results of evaluating renal function of neonates administered with CID.
[FIG. 20] A diagram showing a scheme of a nephron replacement experiment.
[FIG. 21] The results as an immunohistochemically stained image taken 4 days after culturing homozygous fetal kidney, into which rat renal progenitor cells (RPCs) have been injected, in a CID-added culture medium.
[FIG. 22] The results as immunohistochemically stained images taken 14 days after culturing homozygous fetal kidney, into which rat renal progenitor cells have been injected, in a CID-added culture medium.
[FIG. 23(A)] The results as immunohistochemically stained images taken 4 days after culturing heterozygous fetal kidney, into which rat renal progenitor cells have been injected, in a culture medium to which CID and AT406 have been added.
[FIG. 23(B)] The results as immunohistochemically stained images taken 7 days after culturing heterozygous fetal kidney, into which rat renal progenitor cells have been injected, in a culture medium to which CID and AT406 have been added.
[FIGS. 24(A) to 24(C)] The results as immunohistochemically stained images obtained from homozygous fetal kidney cultured after injection of human NPCs derived from human iPS cells.
[FIGS. 25(A) and 25(B)] The results as immunohistochemically stained images obtained one week after injection of human NPCs derived from human iPS cells together with CID into homozygous fetal kidneys.

### DESCRIPTION OF EMBODIMENTS

### <<Non-human animal>>

The present invention provides, in one embodiment, a non-human animal including a system configured to kill a target cell in vivo, in which the target cell includes a chromosome having a cell-specific promoter and an exogenous caspase 9 gene in which expression is inducible downstream of the cell-specific promoter, or a chromosome having a high-expression promoter and an exogenous caspase 9 gene in which expression is inducible downstream of the high-expression promoter.

Examples of the non-human animal include cats, dogs, horses, monkeys, cows, sheep, pigs, goats, rabbits, hamsters, guinea pigs, rats, mice, non-human primates (such as crab-eating macaques or marmosets), and the like. Among these, rodents are preferable. Examples of the rodents include hamsters, guinea pigs, rats, and mice, and the rats and the mice are preferable.

The exogenous caspase 9 gene is not particularly limited as long as it can induce cell death in a target cell through high expression, and it may be a pro-caspase 9 gene, a gene fragment of a caspase 9 gene or a pro-caspase 9 gene, or a modified form thereof. The origin of the exogenous caspase 9 gene is not particularly limited, and it is preferably derived from a mammal, more preferably derived from a mouse or a human, and particularly preferably derived from a human.

The high-expression promoter is not particularly limited as long as it is a strong promoter, and examples thereof include a CMV promoter, an LTR promoter, a PGK promoter, an SV40 promoter, a CK6 promoter, a TTR promoter, a TK promoter, a TRE promoter, an HBV promoter, an hAAT promoter, an LSP promoter, an E2F promoter, an hTERT promoter, a CAG promoter, an EFl-α promoter, and the like, and a CAG promoter is particularly preferable.

Furthermore, it is preferable that the target cell has the exogenous caspase 9 gene in which expression is inducible, in both chromosomes. In the related art, there has been no report on a caspase 9-carrying mouse, but the inventors have increased the probability of obtaining a caspase 9-carrying mouse by controlling the expression of the exogenous caspase 9 gene under a high-expression promoter or by incorporating the introduced gene in both chromosomes.

In addition, in a case where the introduced gene is present in a single allele, it is preferable to use an apoptosis-promoting agent in combination. The apoptosis-promoting agent is not particularly limited, and examples thereof include an IAP inhibitor, a BCL2 inhibitor, TRAIL, a DNA-damaging agent, and the like.

Examples of the IAP inhibitor include AT406, LCL161, GDC-0917, AEG-35156, TL32711, and the like. Examples of the BCL2 inhibitor include 4-[4-[[2-(4-chlorophenyl)-5,5-dimethyl-1-cyclohexen-1-yl]methyl]-1-piperazinyl]-N-[[4-[[(1R)-3-(4-morpholinyl)-1-[(phenylthio)methyl]propyl]amino]-3-[(trifluoromethyl)sulfonyl]phenyl]sulfonyl]benzamide (ABT-263); tetracarcin A; antimycin; gossypol ((-) BL-193); obatoclax; ethyl-2-amino-6-cyclopentyl-4-(1-cyano-2-ethoxy-2-oxoethyl)-4H-chromon-3-carboxylate (HA14-1); oblimersen (G3139); Bak BH3 peptide; (-)-gossypol acetate (AT-101); 4-[4-[(4'-chloro[1,1'-biphenyl]-2-yl)methyl]-1-piperazinyl]-N-[[4-[[(1R)-3-(dimethylamino)-1-[(phenylthio)methyl]propyl]amino]-3-nitrophenyl]sulfonyl]-benzamide (ABT-737, CAS 852808-04-9); navitoclax (ABT-263); and the like.

Examples of the DNA-damaging agent include an alkylating agent, a platinum-based agent, an intercalating agent, a DNA replication inhibitor, and the like. Examples of the DNA alkylating agent include cyclophosphamide, mechlorethamine, uramustine, melphalan, chlorambucil, ifosfamide, carmustine, lomustine, streptozotocin, busulfan, temozolomide, and the like. Examples of the platinum-based agent include cisplatin, carboplatin, oxaliplatin, nedaplatin, satraplatin, triplatin tetranitrate, and the like. Examples of the intercalating agent include doxorubicin, daunorubicin, idarubicin, mitoxantrone, and the like. Examples of the DNA replication inhibitor include irinotecan, topotecan, amsacrine, etoposide, etoposide phosphate, teniposide, and the like.

In the present embodiment, examples of the exogenous caspase 9 gene whose expression is inducible include a gene encoding a fusion protein including a dimerization domain and caspase 9. The fusion protein is activated by binding to a chemical inducer of dimerization (CIDs, protein dimerizer) to dimerize, thereby inducing apoptosis in the cells.

Examples of the dimerization domain include FKBP, FK506, and the like. Examples of the chemical inducer of dimerization include AP20187, AP1903, and the like. Examples of the combination of the multimerization domain and the chemical inducer of dimerization include a combination of a fusion protein of FK506 and caspase 9 with AP1903, and a combination of a fusion protein of FKBP and caspase 9 with AP20187.

In addition, in the present embodiment, examples of the non-human animal including a system configured to induce expression of an exogenous caspase 9 gene include a non-human animal in which the non-human animal includes, in a chromosome, a gene encoding a site-specific recombinase, and has the chromosome containing, upstream of the exogenous caspase 9 gene, a site-specific recombinase recognition sequence, a transcription termination sequence, and a site-specific recombinase recognition sequence in this order from a 5' side.

Examples of the site-specific recombinase include Cre, Flpe, Dre, and the like. Examples of the site-specific enzyme recognition sequence recognized by the site-specific recombinase include loxP, FRT, and rox.

In addition, a fusion protein of a site-specific recombinase and a mutant estrogen receptor (ER) may be used. As an example, the CreERT2 protein is usually present in the cytoplasm, but it migrates to the nucleus by binding to tamoxifen, which is an estrogen derivative, and causes recombination with respect to the loxP sequence. Using this, it is possible to regulate the timing of the Cre-loxP system activation in a tamoxifen-dependent manner.

Specific examples thereof include a system in which a non-human animal that expresses an exogenous caspase 9 in a Cre recombinase activity-dependent manner is mated with a Six2-CreERT2 non-human animal in which a CreERT2 gene is introduced downstream of the promoter of Six2, and a target organ of the obtained offspring is brought into contact with tamoxifen.

The non-human animal that expresses the exogenous caspase 9 in a Crf recombinase activity-dependent manner has a transcriptional stop sequence sandwiched between loxP sequences upstream of the exogenous caspase 9 gene. Therefore, the exogenous caspase 9 is not expressed as it is. However, in a case where the transcription stop sequence sandwiched between the loxP sequences is removed by Cre recombinase, the exogenous caspase 9 is expressed.

In the present embodiment, by appropriately selecting a promoter for expressing the exogenous caspase 9 gene, the exogenous caspase 9 gene can be expressed in an organ-specific manner. The organ is not particularly limited, and examples thereof include the liver, the cornea, the skin, the large intestine, the small intestine, the pancreas, the stomach, the muscle tissue, the heart, the lung, the esophagus, the bone marrow, the kidney, the spleen, the testis, and the ovary, and an organ to be evaluated in an animal experiment is preferable. For example, Six2 is a transcription factor that is specifically expressed in the metanephric mesenchyme. By using the Six2 promoter, it is possible to express the exogenous caspase 9 gene specifically in the metanephric tissue.

For example, in a case where a non-human animal that expresses the exogenous caspase 9 is mated with a Six2-CreERT2 nonhuman animal that specifically expresses CreERT2 in the metanephric mesenchyme, a nonhuman animal that specifically expresses the exogenous caspase 9 in the metanephric tissue appears among the obtained descendants.

The non-human animal of the present embodiment can be used for the development of a non-human animal having an organ made from human cells using an in vivo organ as a scaffold. For example, a human kidney mouse can be produced by injecting exogenous nephron progenitor cells into the kidney developmental domain of a mouse, constructing an exogenous kidney (chimeric kidney) incorporated into the kidney of the host, and then expressing an exogenous caspase 9 gene specifically in the kidney to remove the kidney of the host.

In the construction of the chimeric kidney, examples of the foreign cell include fetal renal cells of a mouse (syngeneic) and a rat (xenogeneic), NPCs induced from mouse ES cells, and NPCs induced from human iPS cells. The host mouse is preferably a fetus or a neonate. In a case where the target is a cell in which the promoter is expressed in the adult, the use of an adult subject is acceptable.

In addition, human stem cells can be transplanted into the brain of a rat to prepare a human brain rat, and the non-human animal of the present embodiment can also be used in neuroscience. In addition, in the human liver mouse, by using the non-human animal of the present embodiment, it is possible to provide a model in which human cells show better engraftment efficiency.

In recent years, cancer treatment in cell therapy such as CAR-T therapy has been actively studied, but there is a concern about the onset of graft-versus-host disease (GVHD) in which transplanted cells attack the host and the possibility that the transplanted cells themselves will become cancerous. In a case where an adverse event occurs after the transplantation of the transplanted cells, cell therapy is safer in a case where the cells can be reset by the inducible cell death. Therefore, the development of a safe and effective inducible cell death system is being pursued. In the development of a target cell inducible death system, the non-human animal of the present embodiment can be used.

In addition, in recent years, research on aging has been actively conducted with aging cells as a target, and the non-human animal of the present embodiment can also be applied to anti-aging research. The inducible death derived from the diphtheria toxin as a conventional technology is an inducible death due to the inhibition of protein synthesis, which resembles necrosis. In necrosis, inflammation or the like is induced after cell death. On the other hand, in the present invention in which Caspase 9 is activated and used, it is considered that the inflammatory response after cell death is weak because apoptosis, which is programmed cell death, is induced instead of necrosis. Therefore, the apoptosis inducible death model of the present invention is suitable for aging studies in which even a small amount of inflammatory response causes noise, instead of the diphtheria toxin model.

### <<Kit>>

The present invention provides, in one embodiment, a kit containing the above-described non-human animal and a chemical inducer of dimerization.

The kit according to the present embodiment includes the above-described configuration described in <<Non-human animal>>. In addition, in a case where the effect of killing the target cell is poor depending on the cell type, it is preferable that the kit includes an apoptosis-promoting agent in addition to the non-human animal and the chemical inducer of dimerization. The details of the apoptosis-promoting agent are the same as the above-described configuration described in <<Non-human animal>>.

### <<Method for producing replacement organ>>

The present invention provides, in one embodiment, a method for producing a replacement organ, including a inducing step of inducing expression of the exogenous caspase 9 gene in the above-described non-human animal, a killing step of killing a target cell of the non-human animal by a caspase 9 protein for which the expression has been induced, and an injection step of injecting a human progenitor cell of the same type as the killed target cell into a site in the non-human animal in which the target cell has been killed.

### [Induction step of expression of exogenous caspase 9 gene]

First, the expression of the exogenous caspase 9 gene is induced in a non-human animal. As described above, since the non-human animal of the present embodiment includes a system that induces expression of an exogenous caspase 9 gene, the expression inducer is administered to the non-human animal. As described above, examples of the expression inducer include the chemical inducer of dimerization and the like. The method for administering the expression inducer is not limited, and examples thereof include oral administration, intravenous administration, intra-arterial administration, intramuscular administration, intradermal administration, subcutaneous administration, intraperitoneal administration, local administration, and the like.

In a case where the administration target is a neonate, subcutaneous administration is preferable, and in a case where the administration target is a pregnant dam carrying fetuses, intraperitoneal administration is preferable. By adjusting the timing of administration of the expression inducer, it is possible to control the number of the residual cells (for example, the number of residual kidney cells).

### [Administration step]

In a case where the effect of killing the target cell is poor depending on the cell type, it is preferable to include an administration step of administering an apoptosis-promoting agent. The administration method is appropriately adjusted depending on the properties of the apoptosis-promoting agent to be used. The details of the apoptosis-promoting agent are the same as the above-described configuration described in <<Non-human animal>>.

### [Killing step]

Apoptosis is induced in cells by inducing the expression of the exogenous caspase 9 gene. In a case where the effect of killing the target cells is poor depending on the cell type, the induction of apoptosis in the cell is achieved by the administration of the apoptosis-promoting agent.

### [Injection step]

Human progenitor cells of the same type as the killed target cells are injected into the site in the non-human animal in which the target cells have been killed. The human progenitor cells may be induced from human iPS cells. The injection step may be performed at the same time as the above-described induction step.

In a case where the replacement ratio is low, a chimeric organ is obtained. In a case where the replacement ratio is high, an organ that can be transplanted to a human can be obtained. The method for producing a replacement organ according to the present embodiment can also be used for producing a congenital kidney disease model.

### [Examples]

Hereinafter, the present invention will be described with reference to Examples, but the present invention is not limited to the following Examples.

### [Experimental Example 1]

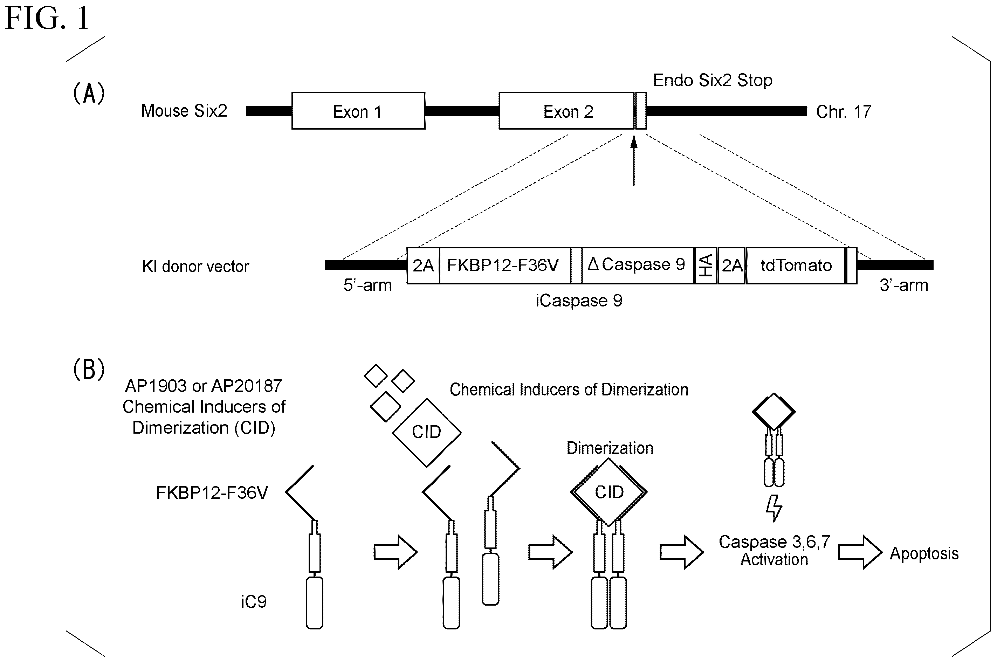

As shown in FIG. 1, a Six2-iCaspase9-tdTomato mouse (hereinafter, Six2-iCaspase9-tdTomato mouse) was prepared by introducing a gene in which Six2 promoter, and a caspase9 gene and tdTomato, which are connected to the downstream of the Six2 promoter, into both alleles in mice.

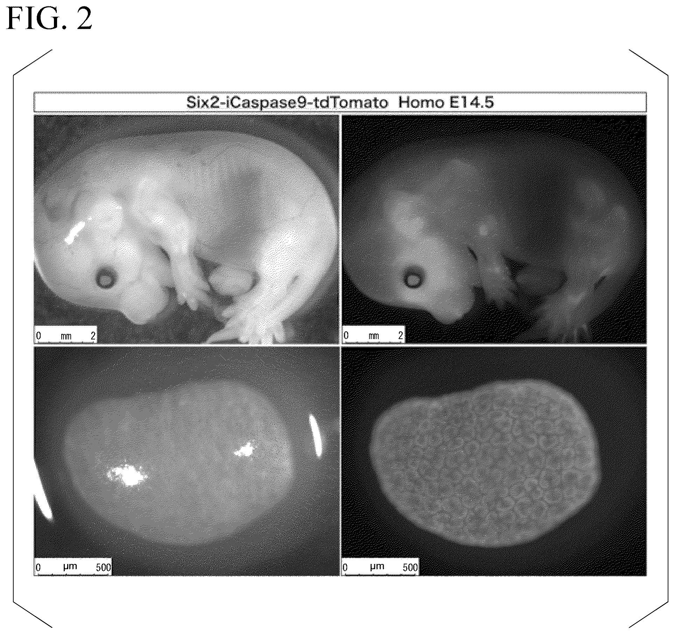

As shown in FIG. 2, the expression of tdTomato was confirmed in Six2-positive nephron progenitor cells in the mouse fetus and the fetal mouse kidney.

### [Experimental Example 2]

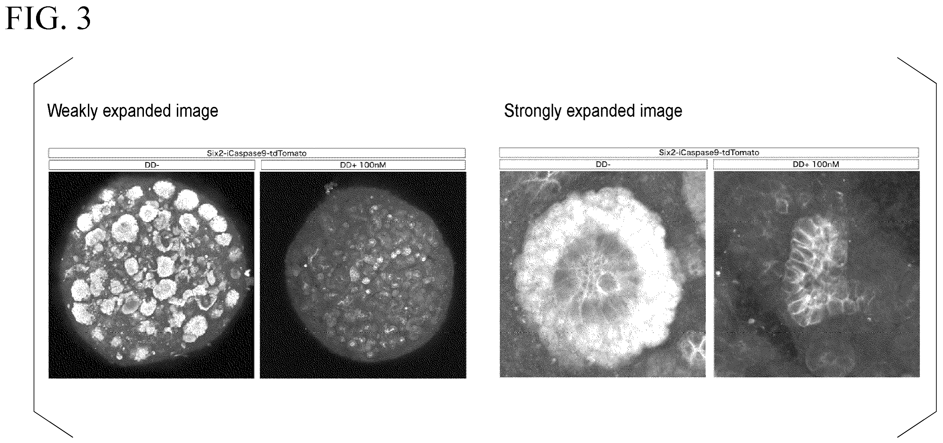

Nephron progenitor cells were isolated from the Six2-iCaspase9-tdTomato mouse prepared in Experimental Example 1, and a kidney organoid was prepared by three-dimensional culture. AP20187 (hereinafter, referred to as CID) was administered to the kidney organoid. FIG. 3 shows an immunostained image of the kidney organoid. It was confirmed that nephron progenitor cells were subjected to apoptosis by the CID administration.

### [Experimental Example 3]

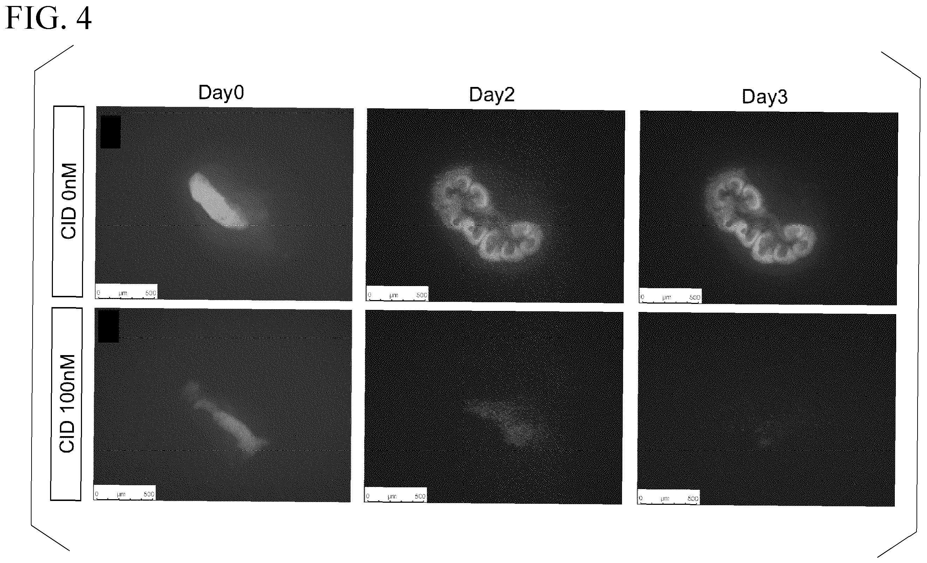

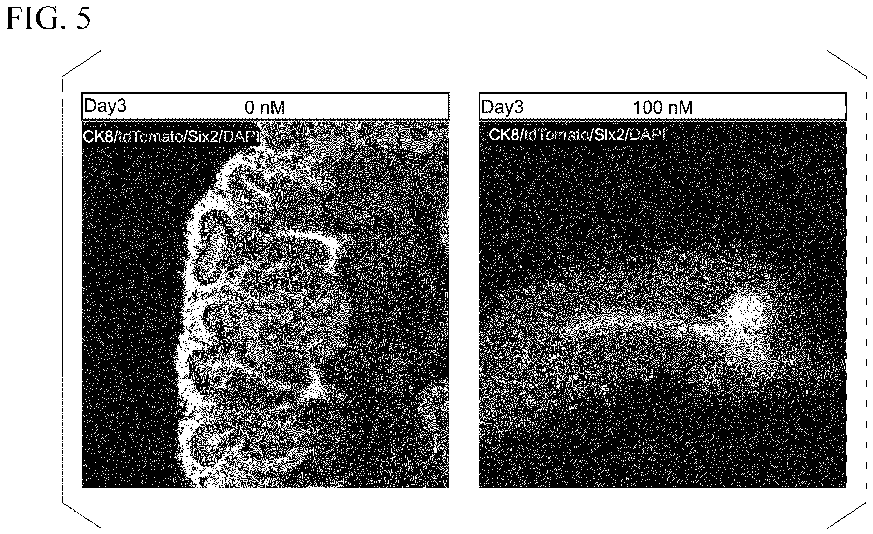

The kidney was excised from an E11 fetus of the Six2-iCaspase9-tdTomato mouse prepared in Experimental Example 1, and organ culture was performed on a Transwell. In the group in which AP20187 (CID) was administered to the culture medium, the expression of tdTomato fluorescence in nephron progenitor cells was reduced on the second day after administration (see FIG. 4). Also in the immunostaining, the removal of Six2-positive nephron progenitor cells was confirmed in a case where CID was administered (see FIG. 5).

### [Experimental Example 4]

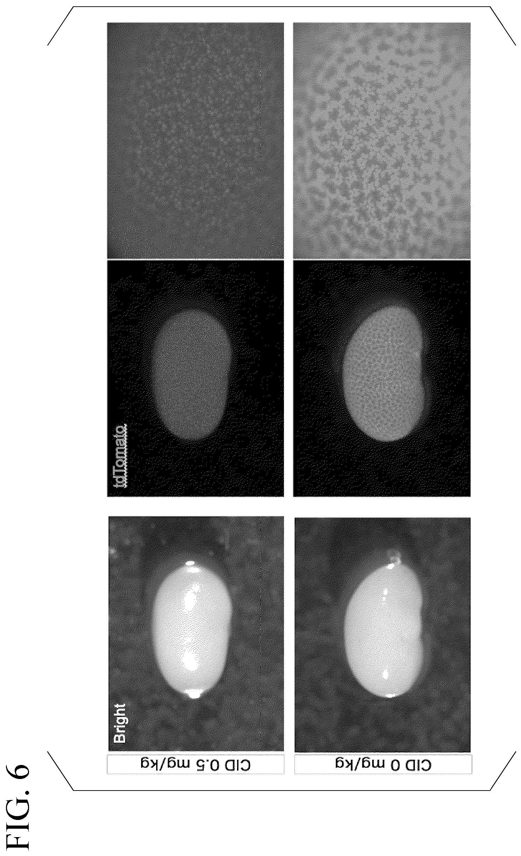

AP20187 (CID) was intraperitoneally administered to neonatal Six2-iCaspase9-tdTomato mice prepared in Experimental Example 1, and the fetal mouse kidneys were collected 48 hours later. FIG. 6 shows bright-field and fluorescence images of the collected fetal mouse kidney. In the CID administration group (upper), it was confirmed that nephron progenitor cells (red) disappeared due to apoptosis.

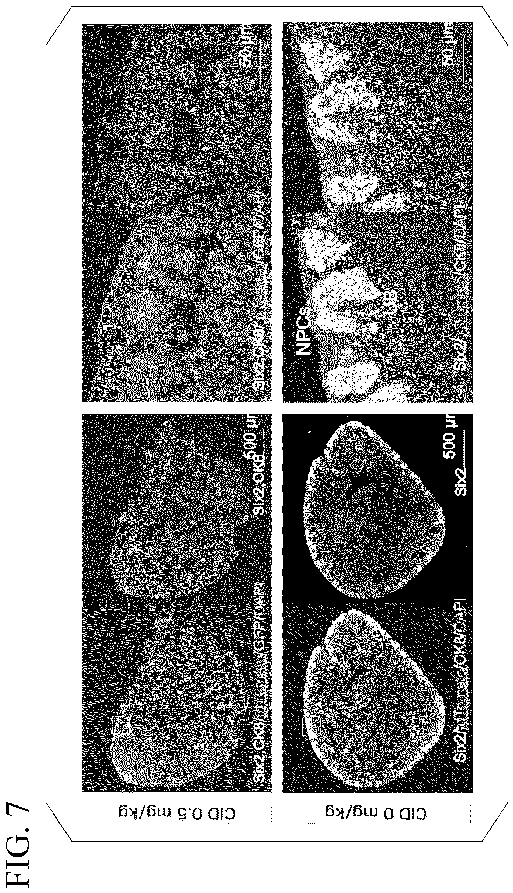

FIG. 7 shows an immunostained image of the same sample. Also in the immunostained image, it was confirmed that the nephron progenitor cells disappeared due to apoptosis since the nephron progenitor cell marker Six2 (white) disappeared in the CID administration group (upper).

### [Experimental Example 5]

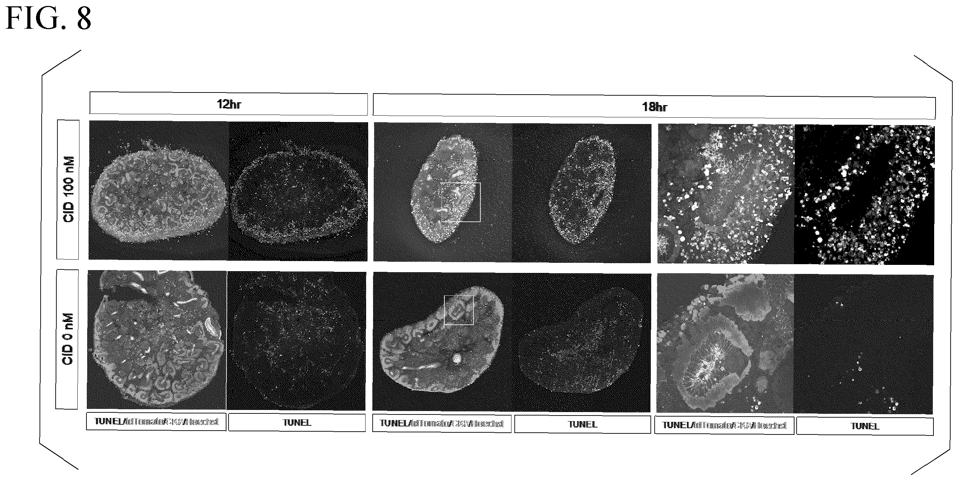

The kidney was excised from an E13 fetus of the Six2-iCaspase9-tdTomato mouse prepared in Experimental Example 1, and organ culture was performed on a Transwell. Apoptosis was evaluated by TUNEL staining after collection at 12 and 18 hours following the administration of 100 nM CID to the culture medium. At 12 hours after administration, TUNEL positivity was observed in co-localization with Six2-positive nephron progenitor cells, indicating early induction of apoptosis (see FIG. 8).

### [Experimental Example 6]

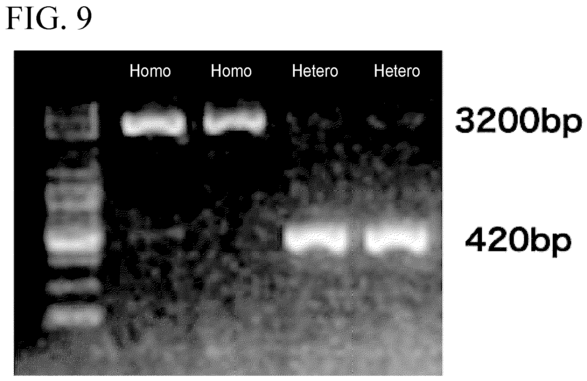

In Experimental Example 1, in a case of preparing a Six2-iCaspase9-tdTomato mouse in which a gene in which a Six2 promoter, and a caspase9 gene and tdTomato, which were connected to the downstream of the Six2 promoter, were introduced into both alleles of a mouse, genotyping was performed on the transgenic mouse. As shown in FIG. 9, mice into which the gene has been introduced in both allelic (homozygous) and single allelic (heterozygous) have been obtained.

### [Experimental Example 7]

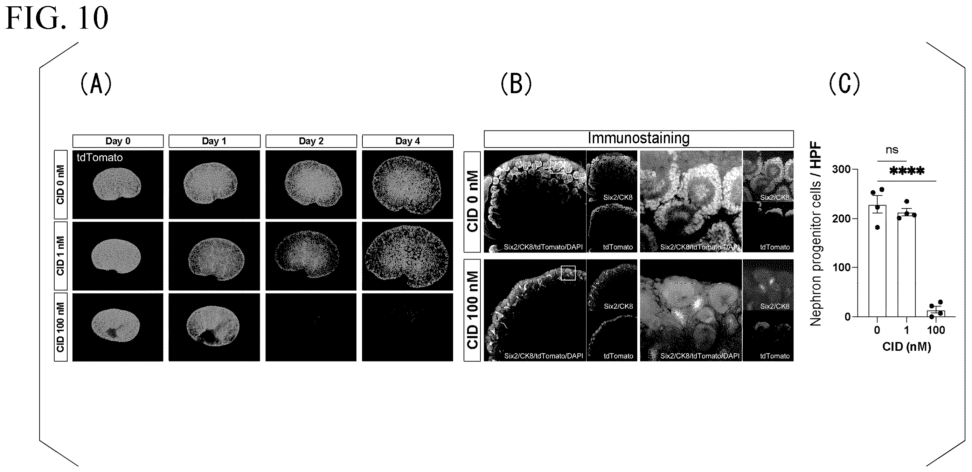

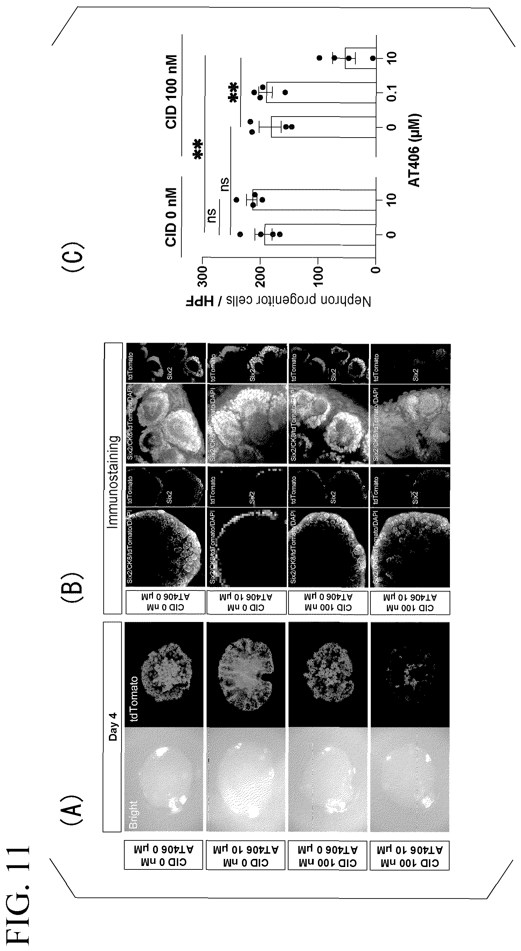

In a case where the kidneys of E13.5 fetuses of both allele knock-in (homozygous) mice were collected, CID was added thereto, and the kidneys were cultured, it was confirmed that 90% or more of NPCs were removed by adding 100 nM of CID (see FIG. 10). On the other hand, even in a case where 100 nM of CID was added to the fetal kidney of a single allele knock-in (heterozygous) animal, the NPC removal was not confirmed (see FIG. 11).

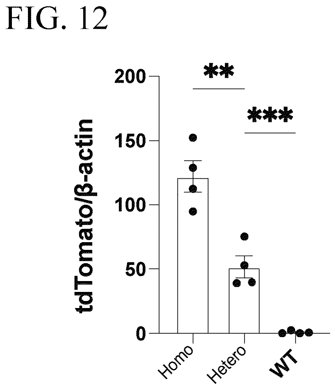

As a difference between homozygous and heterozygous, it is assumed that the expression level of iCaspase9 is different, and in fact, in a case where the protein amount of tdtomato included in the knock-in vector is quantified, the homozygous fetal kidney exhibits an expression level of about 2 times that of heterozygous (see FIG. 12). In the culture of the heterologous fetal kidney, in a case where AT406, which is an XIAP inhibitor, was added as an apoptosis-promoting agent in addition to CID, NPC removal by the addition of 100 nM of CID and 10 µM of AT406 was confirmed. It was also confirmed that NPC removal did not occur in the case of the single administration of AT406, and the NPC removal was due to the action of iCaspase9 (see FIG. 11).

### [Experimental Example 8]

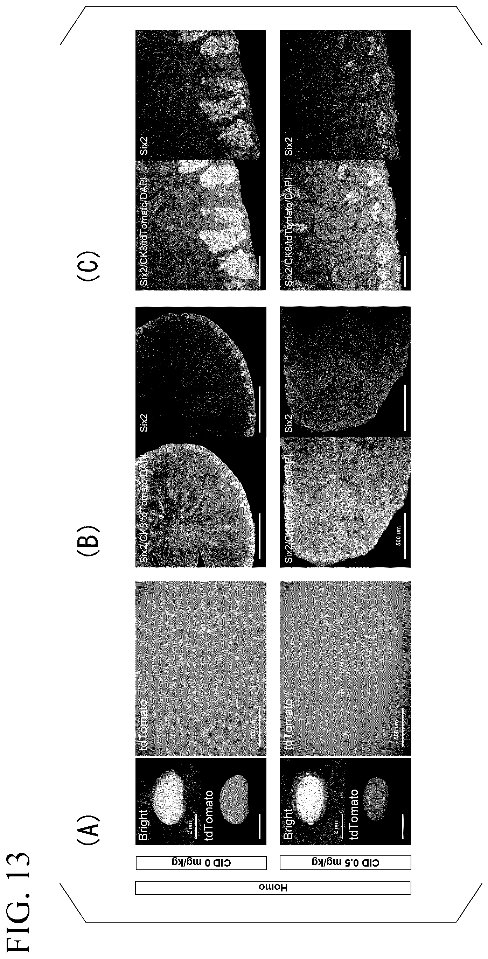

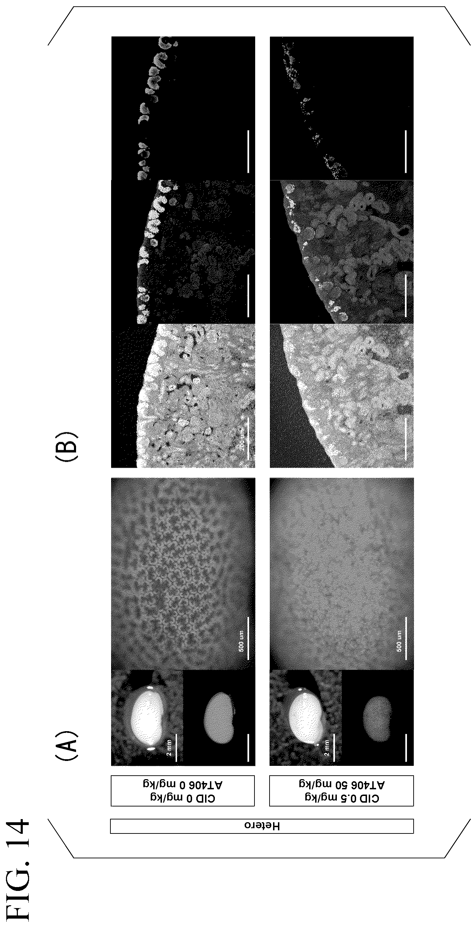

Furthermore, in a case where CID was subcutaneously administered to homozygous neonatal mice, the NPC removal was confirmed (see FIG. 13). In heterozygous neonatal mice, NPC removal does not occur in a case of single administration of CID, but NPC removal could be induced in a case of being used in combination with AT406 (see FIG. 14), and the same results were also confirmed in vivo.

### [Experimental Example 9]

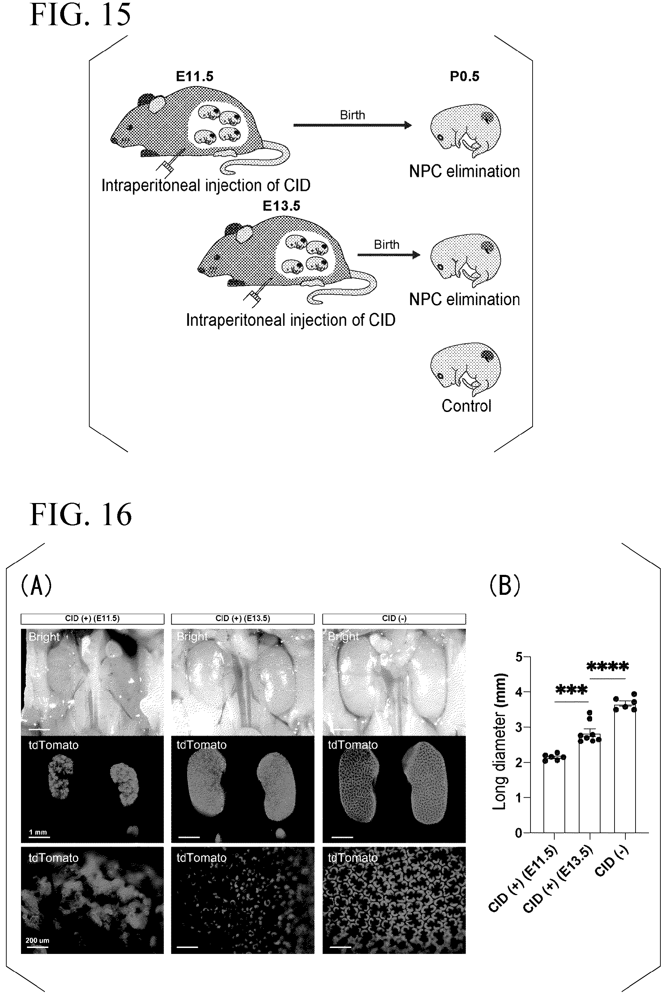

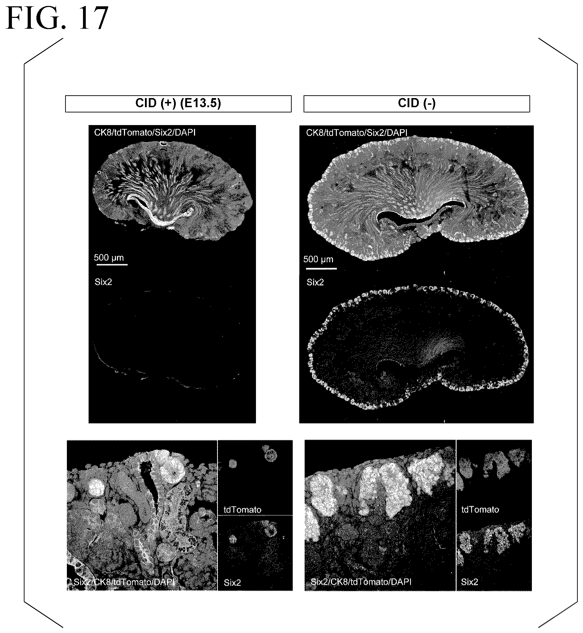

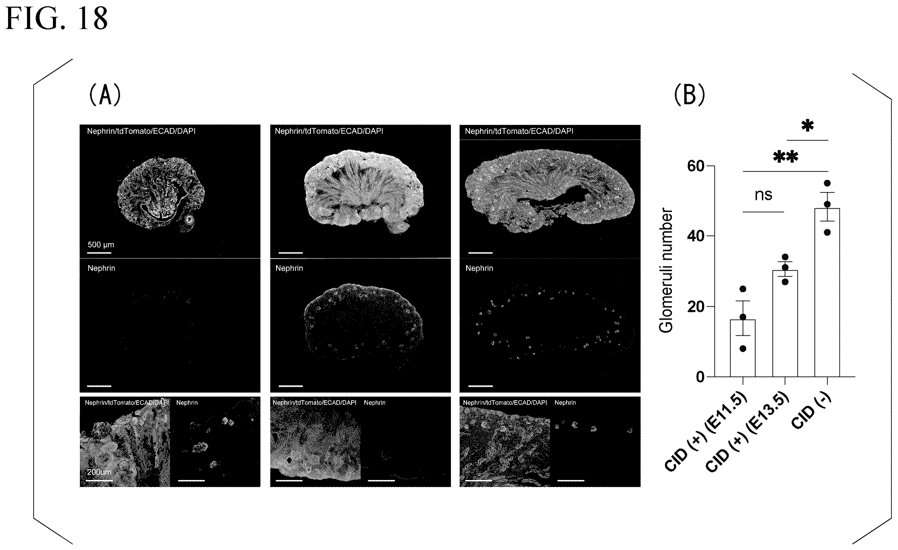

The CID was intraperitoneally administered to a pregnant dam carrying homozygous fetuses (see FIG. 15). As a result, it was confirmed that the kidney size of the neonates (P0) was reduced and the cap mesenchyme structure, which constitutes the nephrogenic niche formed by NPCs, was disrupted (see FIG. 16). In addition, the disappearance of the NPCs was confirmed by immunostaining (see FIG. 17). Furthermore, a decrease in the number of glomeruli was also confirmed (see FIG. 18).

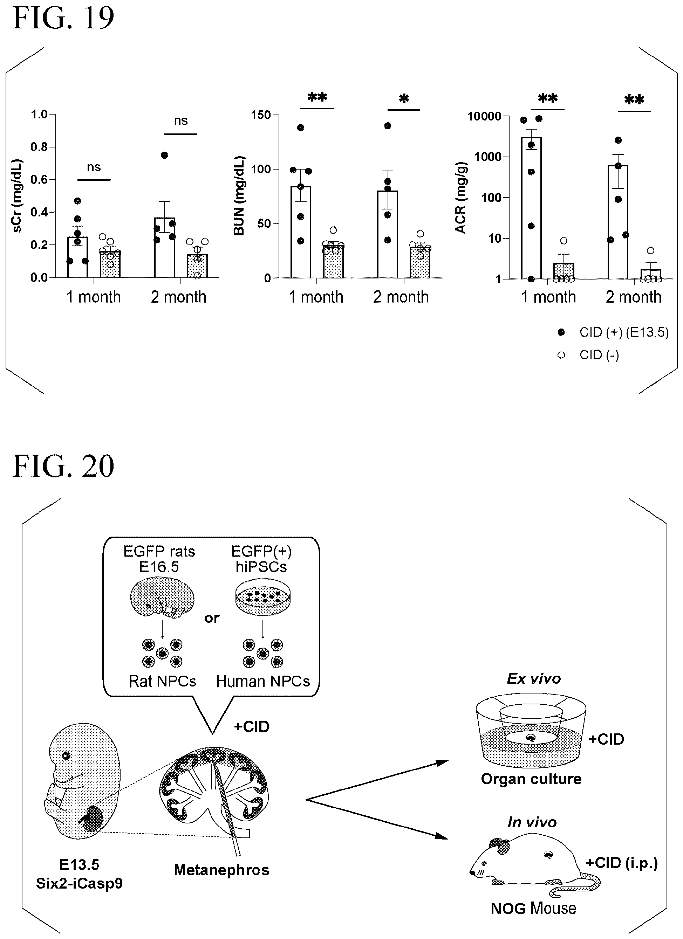

In the administration at the time point of E11.5, animals after birth died due to severe renal failure, but in the administration at the time point of E13.5, the survival of animals was confirmed. In a case where the renal function of the animal at 1 month of age was evaluated, it was confirmed that serum urea nitrogen (BUN) and urinary albumin-to-creatinine ratio (ACR) were significantly increased as compared with the control (see FIG. 19).

### [Experimental Example 10]

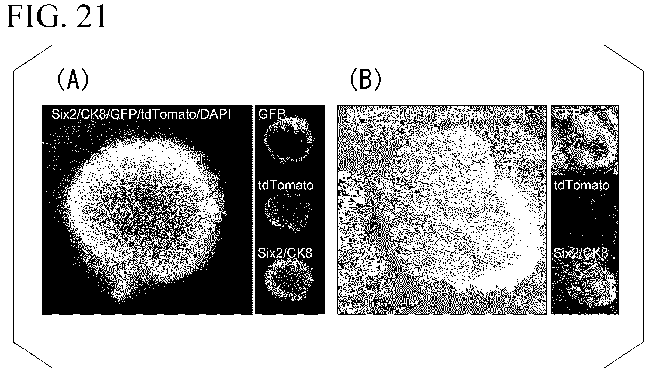

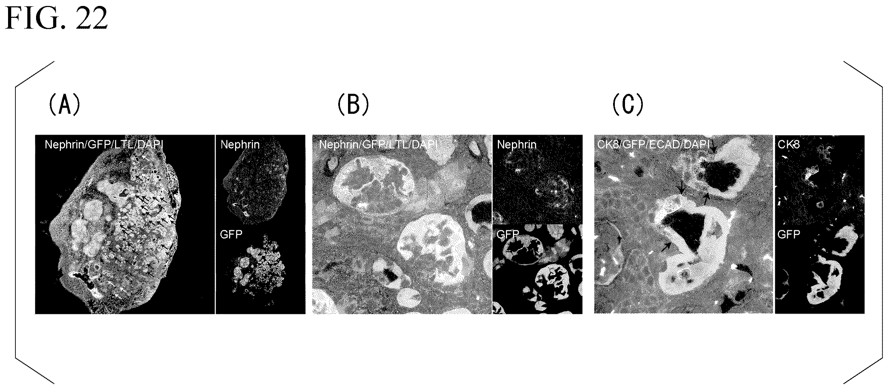

It was verified whether or not the replacement of nephron could be induced by transplanting the exogenous NPC at the same time as the removal of the NPC of the fetal kidney (see FIG. 20). In a case where renal progenitor cells (RPCs) of rats were injected into a homozygous fetal kidney and cultured in a CID-added culture medium, it was confirmed that at the time point of day 4, the renal progenitor cells adhered to the ureteral bud of the host and formed a chimeric cap mesenchyme (see FIG. 21). In addition, in a case where the cells were transplanted into immunodeficient mice after the cell injection and allowed to develop in vivo, it was confirmed that at the time point of day 14, the rat-derived cells differentiated into glomeruli and renal tubules, and approximately 80% of the glomeruli were replaced with rat-derived cells. A rat distal tubule connected to the collecting duct derived from the fetal mouse kidney was also confirmed (see FIG. 22).

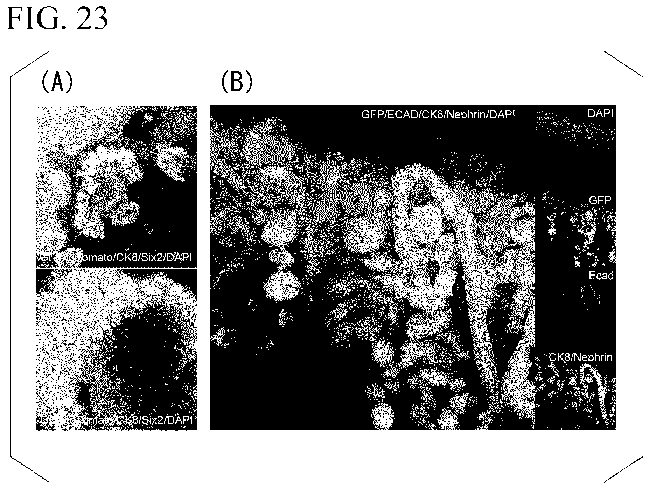

In addition, in the heterologous fetal kidney, in a case where the rat RPCs were injected and cultured in a culture medium to which CID and AT406 were added, it was confirmed that at the time point of day 4, a chimeric cap mesenchyme was formed (see FIG. 23(A)), and at the time point of day 7, a rat distal tubule connected to the mouse collecting duct was formed (see FIG. 23(B)).

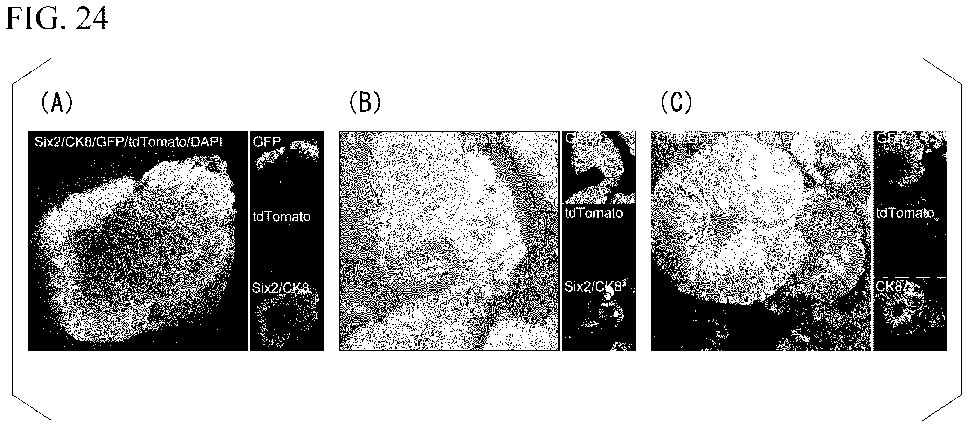

Furthermore, in a case where human NPC induced from human iPS cells were injected into and cultured in a homozygous fetal kidney, it was confirmed that chimeric cap mesenchyme was formed (see FIG. 24(A) and 24(B)), and a part thereof differentiated into renal vesicle (see FIG. 24(C)).

### [Experimental Example 11]

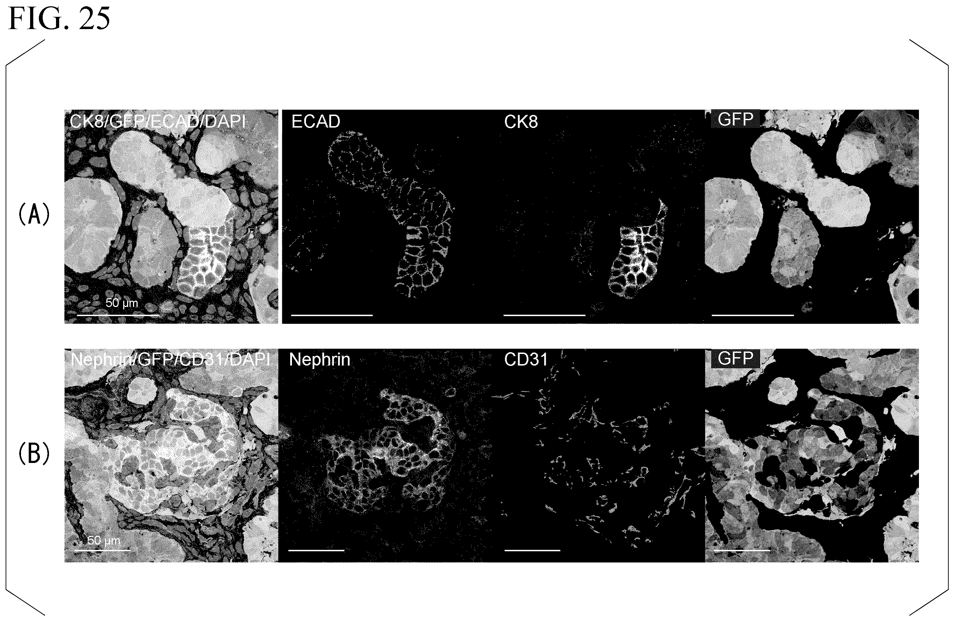

The human NPCs were injected into the fetal kidney of the present mouse together with CID. One week after the injection, the human distal tubule (GFP+, ECAD+) and the collecting duct (GFP-, CK8+) derived from the GFP-negative fetal kidney mouse were connected (see FIG. 25(A)).

It was shown that human-derived glomeruli are also formed (GFP+, Nephrin+), and the maturation is promoted by vascular invasion from immunodeficient mice (GFP-, CD31+) (see FIG. 25(B)).

### INDUSTRIAL APPLICABILITY

According to the present invention, it is possible to provide a non-human animal, a kit, and a method for producing a replacement organ, in which target cells are removed with high efficiency and target cell death is rapidly induced.

## Claims

1. A non-human animal comprising:
a system configured to kill a target cell in vivo,
wherein the target cell includes a chromosome having a cell-specific promoter and an exogenous caspase 9 gene in which expression is inducible downstream of the cell-specific promoter, or a chromosome having a high-expression promoter and an exogenous caspase 9 gene in which expression is inducible downstream of the high-expression promoter.

2. The non-human animal according to Claim 1,
wherein the exogenous caspase 9 gene is a gene encoding a fusion protein including a dimerization domain and caspase 9.

3. The non-human animal according to Claim 1,
wherein the non-human animal includes, in a chromosome, a gene encoding a site-specific recombinase, and
the non-human animal has the chromosome containing, upstream of the exogenous caspase 9 gene, a site-specific recombinase recognition sequence, a transcription termination sequence, and a site-specific recombinase recognition sequence in this order from a 5' side.

4. A kit comprising:
the non-human animal according to Claim 2; and
a chemical inducer of dimerization.

5. The kit according to Claim 4, further comprising:
an apoptosis-promoting agent.

6. A method for producing a replacement organ, comprising:
a inducing step of inducing expression of the exogenous caspase 9 gene in the non-human animal according to any one of Claims 1 to 3;
a killing step of killing a target cell of the non-human animal by a caspase 9 protein for which the expression has been induced; and
an injection step of injecting a human progenitor cell of the same type as the killed target cell into a site in the non-human animal in which the target cell has been killed.

7. The method for producing a replacement organ according to Claim 6, further comprising:
an administration step of administering an apoptosis-promoting agent.
